# EUROPÄISCHE PATENTSCHRIFT

(11) **EP 1 462 432 B1**
(45) Veröffentlichungstag und Bekanntmachung des Hinweises auf die Patenterteilung: **14.08.2013**
(21) Anmeldenummer: 04100258.5
(22) Anmeldetag: 26.01.2004
(51) Int. Cl.: C07C 29/80, C07C 31/12

(54) **Verfahren zur Ausschleusung von 2-Butanol aus tert.-Butanol/Wasser-Gemischen**
Process for removing 2-butanol from tert.-butanol/water mixtures
Procédé d'élimination de 2-butanol d'un mélange tert.-butanol/eau

(30) Priorität: 22.03.2003 DE 10312917
(43) Veröffentlichungstag der Anmeldung: 29.09.2004
(73) Patentinhaber: Evonik Oxeno GmbH, 45772 Marl (DE)
(72) Erfinder: Beckmann, Andreas, Dr., 45657, Recklinghausen (DE); Reusch, Dieter, Dr., 45772, Marl (DE)
(74) Vertreter: Hirsch, Hans-Ludwig

(56) Entgegenhaltungen:
- EP-A1- 0 304 770
- JP-A- 60 260 531
- US-A- 5 985 100
- DATABASE WPI Section Ch, Week 198606 Derwent Publications Ltd., London, GB; Class E17, AN 1986-039691 XP002279550 & JP 60 260531 A (NIPPON ZEON KK) 23. Dezember 1985 (1985-12-23)
- INASHIMA MAKOTO ET AL: "Purification of a crude tert-butyl alcohol", CAPLUS, XP002279771,

## Beschreibung

Die vorliegende Erfindung betrifft ein Verfahren zur Abtrennung von 2-Butanol (im Text auch als sekundäres Butanol oder SBA bezeichnet) aus tert.-Butanol/Wasser-Gemischen, die bei der Spaltung von tert.-Butanol (TBA), insbesondere solchem hergestellt aus technischen C₄-Kohlenwasserstoffgemischen, zu Isobuten und Wasser anfallen.

Eine Abtrennung von SBA aus einem technischen Gemisch, das SBA, TBA und Wasser aufweist, wobei der Massenanteil an Wasser in dem Gemisch sicher größer ist als die Grenzkonzentrationen der beiden Azeotrope TBA/Wasser und SBA/Wasser verbindenden Destillationsgrenzlinie, wobei in einem ersten Schritt aus dem Gemisch mit Hilfe eines Destillationsverfahrens Wasser abgetrennt wird und bei dem in einem zweiten Schritt das Gemisch destillativ in einem SBA aufweisenden Strom und einem überwiegend TBA und Wasser aufweisenden Strom getrennt wird, ist aus EP 0 304 770 A1 bekannt. Isoliert betrachtet, findet es in den in Figur 4 gezeigten Kolonnen 4 und 5 statt.

Bei dem Eingangsstrom 25 des in Figur 4 der EP 0 304 770 A1 gezeigten Prozesses handelt es sich um ein Gemisch aus Wasser, TBA und SBA, was allerdings aufgrund seines dominanten SBA-Anteils nicht dem Oberbegriff des Anspruch 1 entspricht.

Isobuten ist ein Ausgangsstoff für die Herstellung von Butylkautschuk Polyisobutylen, Isobuten-Oligomeren, verzweigten C₅-Aldehyden und C₅-Carbonsäuren. Weiterhin wird es als Alkylierungsmittel und als Zwischenprodukt zur Erzeugung von Peroxiden eingesetzt.

In technischen Strömen liegt Isobuten zusammen mit gesättigten und ungesättigten C₄-Kohlenwasserstoffen vor. Aus diesen Gemischen kann Isobuten wegen der geringen Siedepunktsdifferenz bzw. des sehr geringen Trennfaktors zwischen Isobuten und 1-Buten durch Destillation nicht wirtschaftlich abgetrennt werden. Daher wird Isobuten aus technischen Kohlenwasserstoffgemischen dadurch gewonnen, dass Isobuten zu einem Derivat umgesetzt wird, das sich leicht vom übrig gebliebenden Kohlenwasserstoffgemisch abtrennen lässt, und dass das isolierte Derivat zu Isobuten und Derivatisierungsmittel zurückspaltet wird.

Üblicherweise wird Isobuten aus C₄-Schnitten, beispielsweise der C₄-Fraktion eines Steamcrackers, wie folgt abgetrennt. Nach Entfernung des größten Teils der mehrfach ungesättigten Kohlenwasserstoffe, hauptsächlich Butadien, durch Extraktion(-sdestillation) oder Selektivhydrierung zu linearen Butenen wird das verbleibende Gemisch (Raffinat I oder hydriertes Crack-C₄) mit Alkohol oder Wasser umgesetzt. Bei der Verwendung von Methanol als Alkohol entsteht Methyl-tert.-butylether (MTBE) und bei Einsatz von Wasser tert.-Butanol (TBA). Nach ihrer Abtrennung können beide Produkte in Umkehrung ihrer Bildung zu Isobuten gespalten werden.

Die Spaltung von TBA lässt sich leichter als die Spaltung von MTBE durchführen und ergibt weniger Nebenprodukte und ist somit das bevorzugte Verfahren zur Gewinnung von Isobuten. Bevorzugt wird die Spaltung von TBA in Gegenwart einer Säure in der Gas- oder Flüssigphase unter Teilumsatz von TBA durchgeführt.

Werden zur Herstellung von TBA aus Isobuten Isobuten-haltige Kohlenwasserstoffströme, die auch lineare Butene enthalten, eingesetzt, entsteht in geringen Mengen auch 2-Butanol (SBA).

Dies ist solange nicht weiter kritisch, wie das entstandene Reaktionsgemisch in ein reines TBA oder in ein TBA/Wasser-Azeotrop aufgearbeitet wird. Dabei wird wegen des nur geringen 2-Butanolgehalts im Reaktionsgemisch die maximal zulässige 2-Butanolkonzenzentration von beispielsweise 0,2 Massen-% im TBA oder im TBA/Wasser-Azeotrop nicht überschritten.

Wird das technische TBA oder TBA/Wasser-Azeotrop allerdings unter Teilumsatz in Isobuten und Wasser gespalten, so fällt nach Abtrennung des entstandenen Isobutens ein TBA/Wasser-Gemisch an, in dem das 2-Butanol (SBA) angereichert ist. Dieses Gemisch ist ohne eine Abtrennung von 2-Butanol nicht zur Herstellung von marktgängigen TBA oder TBA/Wasser-Azeotrop geeignet. Ebenfalls ist es nicht zweckmäßig, aus diesem Gemisch Isobuten herzustellen, weil mit zunehmenden 2-Butanolgehalt auch die Konzentration von linearen Butenen im Isobuten steigt, wodurch dessen Spezifikation nicht eingehalten werden kann. Daher ist es notwendig, einen Teil des 2-Butanols unter Vermeidung von TBA-Verlusten auszuschleusen.

Aufgabe der vorliegenden Erfindung war es deshalb ein Verfahren bereitzustellen, mit welchem es möglich ist, SBA aus Gemischen, die SBA, TBA und Wasser aufweisen abzutrennen, ohne dass es zu Verlusten an TBA kommt.

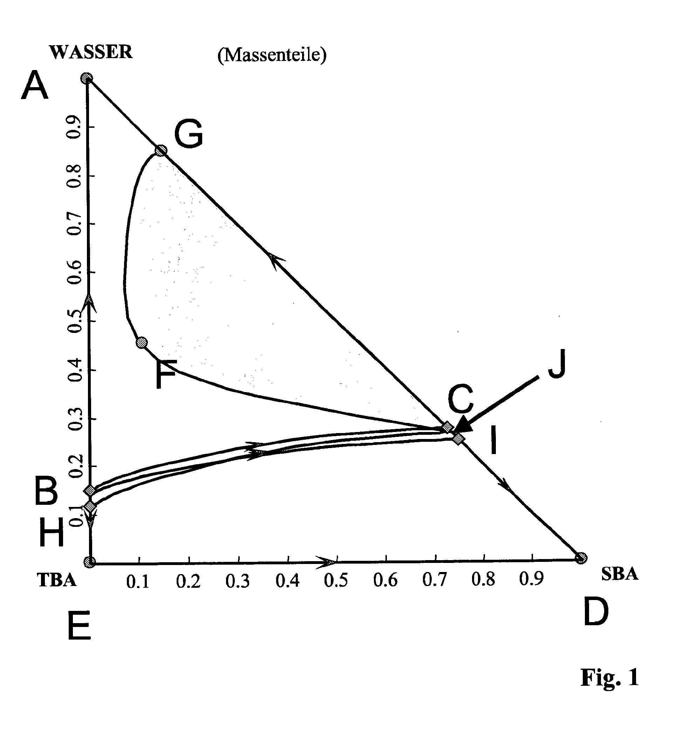

Das Dreistoffgemisch aus SBA, TBA und Wasser ist allerdings destillativ schwierig zu trennen, da in diesem Dreistoffsystem eine Destillationsgrenzlinie (in der Literatur manchmal auch als Grenzdestillationslinie bezeichnet) verläuft, die das binäre Azeotrop Wasser/TBA bei ca. 11 Massen-% Wasser (in der Literatur sind Werte bei Normaldruck von 10 bis 12,5 Massen-% zu finden) (Punkt H in Fig. 1) mit dem binären Azeotrop Wasser/SBA bei ca. 28 Massen-% Wasser (in der Literatur sind Werte bei Normaldruck von 26,7 bis 32 Massen-% zu finden) (Punkt J in Fig. 1) verbindet. Durch diese Destillationsgrenzlinie werden zwei Destillationsfelder getrennt. Kennzeichnend für das obige Dreistoffsystem, dargestellt in Figur 1, sind zwei Destillationsfelder: Destillationsfeld 1 im Bereich A-H-J-A und Destillationsfeld 2 im Bereich H-E-D-J-H. Im Destillationsfeld 1 findet man als Schwersieder Wasser, der Leichtsieder in diesem Bereich ist das TBA/Wasser-Azeotrop und der Mittelsieder das SBA/Wasser-Azeotrop, das nicht in reiner Form abgetrennt werden kann.

Um SBA aus einem TBA-Isobuten-Anlagenverbund auszuschleusen, ist es am wirtschaftlichsten, den SBA-reichsten Strom dazu zu verwenden. Die bei der TBA-Spaltung erhaltenen Ströme weisen aber einen relativ geringen Gehalt an SBA auf. Üblicherweise weisen sie Zusammensetzungen auf, die im Destillationsfeld 1 liegen. Meistens enthalten diese Ströme auch noch geringe Mengen weiterer Stoffe, deren Gegenwart man in diesem Zusammenhang allerdings nicht zu betrachten braucht. Versucht man ein solches Gemisch mit einer Zusammensetzung im Bereich des Destillationsfeldes 1 destillativ aufzuarbeiten, kann man entweder reines Wasser als Schwersieder und ein Gemisch aus SBA/TBA/Wasser als Kopffraktion gewinnen oder aber im Destillat einer Kolonne als leichtestsiedendes Gemisch das TBA/Wasser-Azeotrop und im Sumpf eine höher siedende Mischung aus SBA/TBA/Wasser mit einem hohen Wasseranteil erhalten. Man kann also aus Massenbilanzgründen und aufgrund des ungünstigen Verlaufs der Destillationslinien in keiner Weise den Gehalt an SBA derart erhöhen, dass eine Ausschleusung dieses Stroms wirtschaftlich sinnvoll ist. Auch die im System vorhandene Mischungslücke (siehe Figur 1: C-F-G-C) kann nicht wirtschaftlich für die Trennung der Komponenten oder deren Anreicherung verwendet werden.

Überraschenderweise wurde aber gefunden, dass aus einem Produktionsstrom, der Wasser, SBA und TBA aufweist und dessen Zusammensetzung im Bereich des Destillationsfelds 1 liegt, insbesondere aus einem Produktionsstrom, in dem SBA angereichert ist, SBA praktisch ohne Verluste an TBA abgetrennt werden kann, wenn die Zusammensetzung des Einsatzgemisches mit Hilfe eines ein- oder mehrstufigen Destillationsverfahren derart an Wasser abgereichert wird, dass unter Druckänderung das derart erhaltene Gemisch eine Lage der Zusammensetzung annimmt, die im Bereich des Destillationsfelds 2 zu liegen kommt und somit dieses Gemisch destillativ in SBA und in ein TBA/Wasser-Gemisch aufgetrennt werden kann.

Durch die Druckänderung ändert sich die Lage der Destillationsgrenzlinie im Bereich von B-C bei niedrigem Druck über annähernd H-J (die genaue Lage des Endpunkts liegt zwischen C und I) bei mittleren Drücken nach B-I bei hohem Druck. Somit kann man sich zunächst im Destillationsfeld 1 der Grenze H-J durch Destillation nähern und nach Druckänderung im Destillationsfeld 2, beschrieben durch die Grenze B-C bei niedrigem Druck bzw. B-I bei hohem Druck, im wesentlichen reines SBA abtrennen.

Gegenstand der Erfindung ist demnach ein Verfahren zur Abtrennung von SBA aus einem technischen Gemisch, das SBA, TBA und Wasser aufweist, wobei der Massenanteil an Wasser in diesem Gemisch größer ist als die Grenzkonzentrationen der die beiden Azeotrope TBA/Wasser und SBA/Wasser verbindenden Destillationsgrenzlinie, also das Gemisch hinsichtlich seiner SBA/TBA/Wasser-Zusammensetzung im Bereich des Destillationsfelds 1 liegt, welches dadurch gekennzeichnet ist, dass in einem ersten Schritt aus dem Gemisch mit Hilfe eines Destillationsverfahrens so viel Wasser abgetrennt wird, dass das erhaltene Destillat hinsichtlich seiner SBA/TBA/Wasser-Zusammensetzung bei einem vorgegebenen Druck einen Massenanteil an Wasser aufweist, der gerade eben noch größer ist als die Grenzkonzentration der die beiden Azeotrope TBA/Wasser und SBA/Wasser verbindenden Destillationsgrenzlinie also hinsichtlich seiner SBA/TBA/Wasser-Zusammensetzung im Bereich des Destillationsfeld 1 liegt, und in einem zweiten Schritt anschließend der Druck derart geändert wird, dass das Gemisch hinsichtlich seiner SBA/TBA/Wasser-Zusammensetzung bei einem vorgegebenen Druck einen Massenanteil an Wasser aufweist, der kleiner ist als die Grenzkonzentration der die beiden Azeotrope TBA/Wasser und SBA/Wasser verbindenden Destillationsgrenzlinie bei diesem Druck und bei diesem Druck das Gemisch destillativ in einen SBA aufweisenden Strom und einen überwiegend TBA und Wasser aufweisenden Strom getrennt wird.

Im erfindungsgemäßen Verfahren erfolgt also die SBA-Abtrennung durch eine Kombination von zwei Destillationsschritten, wobei zwischen diesen Schritten der Druck verändert wird.

Das erfindungsgemäße Verfahren basiert auf der Tatsache, dass zur Beschreibung des Zustands eines Systems jeweils Druck, Volumen und Temperatur notwendig sind. Wobei die einzelnen Größen bei Mehrphasensystemen von der Konzentration der einzelnen Komponenten abhängig sind. Die in Fig. 1 dargestellten Punkte H und J gelten in erster Näherung nur für Drücke im Bereich des Normaldrucks. Für höhere Drücke, verschiebt sich die Destillationsgrenzlinie und verbindet die Punkte B und I; für niedrigere Drücke verschiebt sich die Destillationsgrenzlinie und verbindet die Punkte B und C. Durch Druckerhöhung oder Druckemiedrigung kann also bei gleichbleibender Zusammensetzung erreicht werden, dass diese Zusammensetzung im Destillationsfeld 2 liegt und damit SBA destillativ aus dem Gemisch abgetrennt werden kann. Damit dies gelingen kann muss in einem ersten Destillationsschritt die Zusammensetzung des Gemisches derart eingestellt werden, dass es zwar im Destillationsfeld 1 liegt, aber gleichzeitig in dem Feld, welches durch die Verbindung der Punkte B, H, J und C bzw. der Punkte B, H und 1 begrenzt wird.

Durch das erfindungsgemäße Verfahren ist es nun möglich SBA aus Gemischen abzutrennen, die SBA, TBA und Wasser aufweisen, wobei der Massenanteil an Wasser in diesen Gemischen größer ist als die Grenzkonzentrationen der die beiden Azeotrope TBA/Wasser und SBA/Wasser verbindenden Destillationsgrenzlinie, und damit rein destillativ nicht trennbar sind. Durch die Verwendung des erfindungsgemäßen Verfahrens kann auf die Verwendung von Schleppmitteln oder anderen Fremdstoffen verzichtet werden, so dass auf eine aufwändige Abtrennung dieser Hilfsstoffe vermieden werden kann und die Gefahr der Verunreinigung der Produkte durch diese Hilfsstoffe bei der Aufarbeitung ausgeschlossen wird.

Das erfindungsgemäße Verfahren wird nachfolgend beschrieben, ohne dass das Verfahren auf diese Ausführungsformen beschränkt sein soll.

Das erfindungsgemäße Verfahren zur Abtrennung von SBA aus einem technischen Gemisch, das SBA, TBA und Wasser aufweist, wobei der Massenanteil an Wasser in diesem Gemisch größer ist als die Grenzkonzentrationen der die beiden Azeotrope TBA/Wasser und SBA/Wasser verbindenden Destillationsgrenzlinie, also das Gemisch hinsichtlich seiner SBA/TBA/Wasser-Zusammensetzung im Bereich des Destillationsfelds 1 liegt, welches dadurch gekennzeichnet ist, dass in einem ersten Schritt aus dem Gemisch mit Hilfe eines Destillationsverfahrens so viel Wasser abgetrennt wird, dass das erhaltene Destillat hinsichtlich seiner SBA/TBA/Wasser-Zusammensetzung bei einem vorgegebenen Druck einen Massenanteil an Wasser aufweist, der gerade eben noch größer ist als die Grenzkonzentration der die beiden Azeotrope TBA/Wasser und SBA/Wasser verbindenden Destillationsgrenzlinie also hinsichtlich seiner SBA/TBA/Wasser-Zusammensetzung im Bereich des Destillationsfeld 1 liegt, und in einem zweiten Schritt anschließend der Druck derart geändert wird, dass das Gemisch hinsichtlich seiner SBA/TBA/Wasser-Zusammensetzung bei einem vorgegebenen Druck einen Massenanteil an Wasser aufweist, der kleiner ist als die Grenzkonzentration der die beiden Azeotrope TBA/Wasser und SBA/Wasser verbindenden Destillationsgrenzlinie bei diesem Druck und bei diesem Druck das Gemisch destillativ in einen SBA aufweisenden Strom und einen überwiegend TBA und Wasser aufweisenden Strom getrennt wird. Durch die Änderung des Druckes wird erreicht, dass die Destillationsgrenzlinie derart verschoben wird, dass das im ersten Schritt erhaltene Gemisch nach der Druckänderung eine Zusammensetzung aufweist, die im Destillationsfeld 2 liegt.

Die Abtrennung des Wassers im ersten Schritt des erfindungsgemäßen Verfahrens kann in einem ein- oder mehrstufigen, insbesondere in einem ein- oder zweistufigen Destillationsverfahren erfolgen. Vorzugsweise erfolgt die Abtrennung des Wassers im ersten Schritt mittels einer Destillationskolonne.

Es kann vorteilhaft sein, wenn das Destillationsverfahren im ersten Schritt bei einem Druck von 0,05 bis 15 bar_{abs}. (bara) durchgeführt wird. Nur das TBA/Wasser-Azeotrop zeigt ein Minimum der Wasser-Konzentration bei steigendem Druck (bei ca. 1 bar), beim SBA/Wasser-Azeotrop nimmt die Wasser-Konzentration bei steigendem Druck stetig ab. Vorzugsweise wird daher das Destillationsverfahren im ersten Schritt bei einem Druck von 0,5 bis 5 bara, insbesondere von 0,6 bis 2 bara durchgeführt. Der Druck, bei dem das Destillationsverfahren des zweiten Schrittes durchgeführt wird, ist vorzugsweise um mindestens den Faktor 2, besonders bevorzugt um mindestens den Faktor 3 größer als im ersten Schritt. Bei der Durchführung des Verfahrens unter Druckemiedrigung ist der Druck im ersten Schritt vorzugsweise um mindestens den Faktor 2, besonders bevorzugt um mindestens den Faktor 3 größer als im zweiten Schritt.

Wird das Destillationsverfahren im ersten Schritt bei einem Druck von 0,7 bis 5 bara durchgeführt, so hat es sich als vorteilhaft erwiesen, wenn der Druck im zweiten Schritt durch Druckerniedrigung auf 0,05 bis kleiner 0,7 bara eingestellt wird und die destillative Abtrennung des SBA bei diesem Druck erfolgt oder wenn der Druck im zweiten Schritt durch Druckerhöhung auf größer 5 bis 15 bara eingestellt wird und die destillative Abtrennung des SBA bei diesem Druck erfolgt. Insbesondere im Falle von SBA-Konzentrationen (im Bereich von 0,001 bis 30 Massen-%) kann der Druckbereich auch derart geändert werden, dass der erste Verfahrensschritt bei einem Druck von 0,4 bis 5 bara und der zweite Verfahrenschritt bei einem Druck von 5 bis 15 bara, insbesondere von 5 bis 10 bara, durchgeführt wird, wobei sich die Druckverhältnisse wiederum mindestens um den Faktor 2 unterscheiden sollten. Dies wird dadurch möglich, dass sich der Endpunkt B bzw. H der Grenze der Destillationsfelder 1 und 2 bei moderaten Drücken (von 0,5 bis 5 bara, insbesondere von 0,6 bis 2 bara) nahe Punkt H liegt und bei Drücken kleiner 0,5 bara und größer 5 bara nahe Punkt B liegt. Der Endpunkt C wandert von niedrigen zu hohen Drücken stetig in Richtung Endpunkt I.

Das als Rückstand im Destillationsverfahren des ersten Schrittes abgetrennte Wasser weist vorzugsweise einen Gehalt an organischen Inhaltsstoffen von kleiner 10 Massen-%, bevorzugt kleiner 5 Massen-% und ganz besonders bevorzugt von 3 bis 0,05 Massen-%, insbesondere an TBA auf. Es können auch kleinere Werte bis zu 0,001 Massen-% erreicht werden, wobei dies meistens nicht erforderlich und nicht wirtschaftlich sinnvoll ist. Aus dem wässrigen Rückstand können TBA und SBA zusammen aufkonzentriert und von reinem bzw. nahezu reinem Wasser abgetrennt werden. Diese Abtrennung kann z.B. rein destillativ erfolgen, da ein Gemisch mit einer Zusammensetzung im Bereich des Destillationsfeldes 1 vorliegt und durch Destillation reines bzw. nahezu reines Wasser als Schwersieder und ein Gemisch aus SBA/TBA/Wasser als Kopffraktion gewonnen werden kann. Das Gemisch aus der Kopffraktion kann ganz oder nach Ausschleusung eines Teilstroms partiell in den ersten Schritt des erfindungsgemäßen Verfahrens erneuten Abtrennung von Wasser zurückgeführt werden.

Optional kann der Rückstand ganz oder teilweise direkt verwendet werden, beispielsweise als Prozesswasser in einer Anlage zur TBA-Synthese.

Bei dem Destillationsverfahren im zweiten Schritt des erfindungsgemäßen Verfahrens fällt ein SBA-haltiger Strom als Sumpfprodukt an, der 2-Butanol und gegebenenfalls Hochsieder aufweist. Der Gehalt an TBA in dieser 2-Butanol aufweisende SBA-haltigen Strom, die im zweiten Schritt destillativ aus dem im ersten Schritt erhaltenen Destillat abgetrennt wurde, beträgt vorzugsweise kleiner 2 Massen-%, bevorzugt kleiner 1,7 Massen-%. Als Kopfprodukt wird ein TBA und Wasser aufweisender Strom, der gegebenenfalls noch Leichtsieder aufweisen kann, abgezogen. Der Gehalt an 2-Butanol im Kopfprodukt beträgt vorzugsweise kleiner 4 Massen-%, insbesondere kleiner 3 Massen-%. Im Sumpf der Kolonne lässt sich 2-Butanol ohne oder nahezu ohne Hochsieder gewinnen, indem das 2-Butanol aus der Dampfphase des Verdampfers oder dampfförmig oder flüssig als Seitenstrom im Abtriebsteil der Kolonne abgezogen wird. Ein Teil des im zweiten Schritt als Kopfprodukt erhaltenen TBA und Wasser aufweisenden Stroms kann mit dem im ersten Schritt erhaltenen Destillat vermischt werden und als Zulauf in die destillative Auftrennung des zweiten Schritts eingesetzt werden.

Die mit dem erfindungsgemäßen Verfahren erhaltenen aus dem Gemisch abgetrennten TBA-Fraktionen, die als Destillat im zweiten Schritt des Verfahrens anfallen, können für die bekannten Zwecke verwandt werden. Beispielsweise können sie als Ausgangsmaterial für die Herstellung von Isobuten dienen. Gegebenenfalls darin enthaltene Leichtsieder können destillativ abgetrennt werden.

Das abgetrennte 2-Butanol kann für die üblichen technischen Anwendungen genutzt werden. So kann es z.B. als Vorstufe für Methylethylketon, als Lösemittel für Lacke und Harze, als Bestandteil von Bremsflüssigkeiten sowie als Bestandteil von Reinigungsmitteln eingesetzt werden. Darüber hinaus fmdet es Verwendung bei der Herstellung von Duftstoffen, Farbstoffen und Benetzungsmitteln.

Die destillativen Auftrennungen von bei dem erfindungsgemäßen Verfahren anfallenden Stoffströmen, insbesondere vom Destillat aus dem ersten Verfahrensschritt, kann in ein oder mehreren Kolonnen mit Einbauten, die aus Böden, rotierenden Einbauten, ungeordneten und/oder geordneten Packungen bestehen, durchgeführt werden. Vorzugsweise erfolgt sowohl die Durchführung des Destillationsverfahrens gemäß dem ersten Schritt als auch die Durchführung des Destillationsverfahrens gemäß dem zweiten Schritt jeweils in einer einzigen Kolonne pro Schritt.

Bei den Kolonnenböden können folgende Typen zum Einsatz kommen:
Böden mit Bohrungen oder Schlitzen in der Bodenplatte.
Böden mit Hälsen oder Kaminen, die von Glocken, Kappen oder Hauben überdeckt sind.
Böden mit Bohrungen in der Bodenplatte, die von beweglichen Ventilen überdeckt sind.
Böden mit Sonderkonstruktionen.

In Kolonnen mit rotierenden Einbauten wird der Rücklauf entweder durch rotierende Trichter versprüht oder mit Hilfe eines Rotors als Film auf einer beheizten Rohrwand ausgebreitet.

In dem erfindungsgemäßen Verfahren verwendete Kolonnen können regellose Schüttungen mit verschiedenen Füllkörpern aufweisen. Sie können aus fast allen Werkstoffen - Stahl, Edelstahl, Kupfer, Kohlenstoff, Steingut, Porzellan, Glas, Kunststoffen usw. - und in verschiedenen Formen - Kugeln, Ringen mit glatten oder profilierten Oberflächen, Ringen mit Innenstegen oder Wanddurchbrüchen, Drahtnetzringen, Sattelkörper und Spiralen - bestehen.

Packungen mit regelmäßiger Geometrie können z.B. aus Blechen oder Geweben aus Metall oder Kunststoff bestehen. Beispiele solcher Packungen sind Sulzer Gewebepackungen BX, Sulzer Lamellenpackungen Mellapak aus Metallblech, Hochleistungspackungen wie MellapakPlus, Strukturpackungen von Sulzer (Optiflow), Montz (BSH) und Kühni (Rombopak).

Die bei der Vorentwässerung des technischen Gemisches gemäß des ersten Schrittes des erfindungsgemäßen Verfahrens eingesetzte Kolonne weist bevorzugt eine theoretische Trennstufenzahl von 3 bis 50, insbesondere eine von 6 bis 40 auf. Der Zulaufboden hängt von der Zusammensetzung des Gemischs im Destillationsfeld 1 ab. Die Einspeisung des Zulaufs erfolgt vorzugsweise auf den, von oben gezählten, 2. bis 45. theoretischen Boden, insbesondere auf den 3. bis 35 theoretischen Boden (theoretische Böden entsprechen in diesem Zusammenhang den theoretischen Stufen).

Die Destillation im zweiten Schritt des erfindungsgemäßen Verfahrens, bei dem das SBA abgetrennt wird, wird vorzugsweise in einer Kolonne durchgeführt, die eine theoretische Trennstufenzahl von 5 bis 70, insbesondere eine von 10 bis 60 aufweist. Der Zulaufboden hängt wiederum von der Zusammensetzung des Gemisches ab und erfolgt bevorzugt auf den, von oben gezählten, 2. bis 55. theoretischen Boden, insbesondere auf den 3. bis 35.

Der Betriebsdruck der Kolonnen zur Durchführung des ersten und zweiten Verfahrensschrittes beträgt vorzugsweise von 0,05 und 15 bar_{abs} (bara), wobei der Druck in der Kolonne zur Durchführung des zweiten Verfahrensschrittes wie oben beschrieben mehr oder weniger beträgt als der Druck in der Kolonne zur Durchführung des ersten Verfahrensschrittes.

Mit dem erfindungsgemäßen Verfahren kann 2-Butanol ohne Verluste von TBA aus beliebigen ternären Gemischen aus TBA, SBA und Wasser, die im Destillationsfeld 1 liegen, abgetrennt werden. Dies gelingt selbst dann noch, wenn die Gemische zusätzlich bis zu 5 Massen-% Hochsieder (wie z.B. aus Oligomerisierung von Isobuten hervorgegangene C₈- oder C₁₂-Kohlenwasserstoffe, C₈-Alkohole) und/oder Leichtsieder (wie z.B. Isobuten oder andere C₄-Kohlenwasserstoffe) aufweisen. Mittels des erfindungsgemäßen Verfahrens kann also 2-Butanol, insbesondere 2-Butanol-Gemische, welche einen Gehalt an tert.-Butanol von vorzugsweise kleiner 2, bevorzugt kleiner 1,7 Massen-% aufweisen, hergestellt werden.

Im erfindungsgemäßen Verfahren werden insbesondere TBA-Ströme, in denen 2-Butanol angereichert ist, aus Anlagen, in denen Isobuten aus TBA durch Wasserabspaltung hergestellt wird, eingesetzt. Diese Ströme enthalten meistens C₄-Kohlenwasserstoffe und Folgeprodukte von C₄-Olefinen als weitere Komponenten.

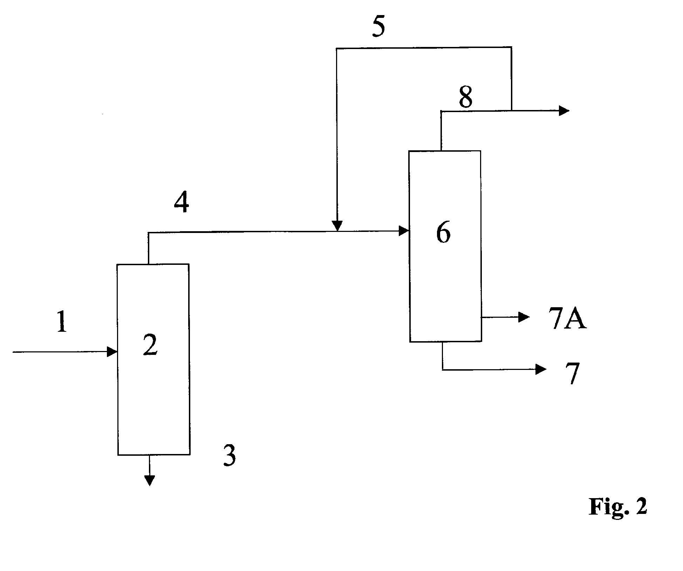

Das Blockschema einer Anlage, in der das erfindungsgemäße Verfahren durchgeführt werden kann, zeigt Fig. 2. Das Einsatzstoffgemisch (1) wird zunächst in einer Kolonne (2) destillativ derart aufgearbeitet, dass im Sumpf der Kolonne ein wasserreicher Strom (3) ausgeschleust wird. Der auf diese Weise vorentwässerte Destillatstrom (4) wird nun optional mit einem Teil (5) des Destillats (8) aus Kolonne (6) gemischt und in die Kolonne (6) eingeleitet. In dieser erfolgt eine Trennung des Gemisches (4) in ein Sumpfprodukt (7) mit dem abzutrennenden 2-Butanol und in ein Kopfprodukt (8), das TBA, Wasser und gegebenenfalls Leichtsieder enthält. Die Kolonne (6) wird hierbei erfindungsgemäß bei einem anderen Druck als Kolonne (2) betrieben. Um 2-Butanol mit einem geringen Anteil an Hochsiedem zu gewinnen, kann man das Produkt aus der Dampfphase des Verdampfers der Kolonne (6) oder dampfförmig oder flüssig als Seitenstrom (7A) im Abtriebsteil der Kolonne (6) abziehen. Der Destillatstrom (8) kann ganz oder teilweise direkt als Zulauf im Anlagenteil einer TBA-Spaltung wieder eingesetzt werden.

Der Zulauf zur Kolonne (6) befindet sich hinsichtlich seines Wassergehaltes bei dem in Kolonne (6) angewandten Druck im Destillationsfeld 2, bei dem in Kolonne (2) angewandten Druck im Destillationsfeld 1. Der Wassergehalt beträgt bezogen auf das Dreikomponentensystem SBA/TBA/Wasser weniger als durch Grenze B-C beschrieben. Weiterhin kann dieses Gemisch bis zu 5, insbesondere bis zu 3, ganz besonders bis zu 2,5 Massen-% an weiteren Stoffen, beispielsweise C₈-Olefinen oder -Alkoholen, enthalten.

Gewöhnliche Bauteile wie Pumpen, Verdichter, Ventile, Wärmetauscher und Verdampfer sind in den Blockschaltbildern nicht dargestellt, jedoch selbstverständliche Bauteile einer Anlage.

Das folgende Beispiel soll die Erfindung erläutern, ohne die Anwendungsbreite einzuschränken, die sich aus der Beschreibung und den Patentansprüchen ergibt.

### Beispiel

Die Abtrennung von SBA erfolgte in einer nach Fig. 2 realisierten Anlage, mit der Besonderheit, dass Strom (5) und (7A) entfielen. Der Kolonnendurchmesser der Kolonne (2) betrug dabei 50 mm. Es wurde eine Metall-Destillations-Packung mit 12 theoretischen Stufen installiert, der Zulauf erfolgte auf der, von oben gezählten, 7. theoretischen Stufe. Der Kolonnendurchmesser der Kolonne (6) betrug ebenfalls 50 mm. Es wurde eine Metall-Destillations-Packung mit 20 theoretischen Stufen installiert, der Zulauf erfolgte auf der, von oben gezählten, 6. theoretischen Stufe. Der Zulauf (1) wurde aus der großtechnischen Anlage entnommen und für die Versuche verwendet. Die Stromnummern in der folgenden Tabelle 1 sind die gleichen wie in Figur 2. Das Destillat (4) der Kolonne (2) wurde gesammelt und zum Teil als Zulauf zur zweiten Kolonne (6) eingesetzt. Komponenten mit einer Konzentration kleiner als 0,1 Massen-% im Gemisch sind in der Regel nicht in der Tabelle aufgeführt.

**Tabelle 1:**

| Stromnummer | Strombezeichnung | Massenfluss [kg/h] | Stromzusammensetzung in Massen-% |
|---|---|---|---|
| 1 | Frisch-Zulauf | 1,80 | 63,5 % Wasser 30,2 % TBA 4,5 % 2-Butanol 1,7 % C₈-Alkohol 0,1 % sonstige Stoffe |
| 3 | Abwasser | 1,03 | 96,9 % Wasser 0,1 % TBA 0,1 % 2-Butanol 2,9 % C₈-Alkohol |
| 4 | Vorentwässertes Gemisch, Destillat der Kolonne (2) | 0,77 | 18,9 % Wasser 70,3 % TBA 10,4 % 2-Butanol 0,1 % C₈-Alkohol 0,3 % sonstige Stoffe |
| 4 | Vorentwässertes Gemisch, Zulauf zur Kolonne (6) | 0,40 | 18,9 % Wasser 70,3 % TBA 10,4 % 2-Butanol 0,1 % C8-Alkohol 0,3 % sonstige Stoffe |
| 5 | Rückführungsstrom | entfällt | |
| 7 | Sumpf der Kolonne (6) | 0,03 | 26,3 % Wasser 1,6 % TBA 71,1 % 2-Butanol 0,9 % C₈-Alkohol 0,1 % sonstige Stoffe |
| 7A | Seitenstromentnahme der Kolonne (6) | entfällt | |
| 8 | Destillat der Kolonne (6) | 0,37 | 18,2 % Wasser 76,7 % TBA 4,8 % 2-Butanol 0,3 % sonstige Stoffe |

Die Kolonne (2) wurde bei 1 bara mit einem Rücklaufverhältnis von 3,5 betrieben. Kolonne (6) wurde bei 0,1 bara mit einem Rücklaufverhältnis von 15 betrieben.

Es ist deutlich zu erkennen, dass es mit dem erfindungsgemäßen Verfahren einfach möglich ist, Gemische, die TBA, SBA und Wasser in einer Zusammensetzung aufweisen, die im Destillationsfeld 1 liegt, der Art zu trennen, dass eine SBA-Fraktion erhalten wird, die weniger als 2 Massen-% TBA aufweist. Durch das erfindungsgemäße Verfahren wird also die Aufgabe der vorliegenden Erfindung gelöst, nämlich die Bereitstellung eines Verfahrens, mit dem SBA aus TBA, SBA und Wasser aufweisenden Gemischen ohne wesentliche Verluste an TBA abgetrennt werden kann.

## Patentansprüche

1. Verfahren zur Abtrennung von 2-Butanol (SBA) aus einem technischen Gemisch, das 2-Butanol, tert.-Butanol (TBA) und Wasser aufweist, wobei der Massenanteil an Wasser in dem Gemisch größer ist als die Grenzkonzentrationen der die beiden Azeotrope TBA/Wasser und BA/Wasser verbindenden Destillationsgrenzlinie,
**dadurch gekennzeichnet,**
**dass** in einem ersten Schritt aus dem Gemisch mit Hilfe eines Destillationsverfahrens so viel Wasser abgetrennt wird, dass das erhaltene Destillat hinsichtlich seiner SBA/TBA/Wasser-Zusammensetzung bei einem vorgegebenen Druck einen Massenanteil an Wasser aufweist, der gerade eben noch größer ist als die Grenzkonzentration der die beiden Azeotrope TBA/Wasser und SBA/Wasser verbindenden Destillationsgrenzlinie, und in einem zweiten Schritt anschließend der Druck derart geändert wird, dass das Gemisch hinsichtlich seiner SBA/TBA/Wasser-Zusammensetzung bei einem vorgegebenen Druck einen Massenanteil an Wasser aufweist, der kleiner ist als die Grenzkonzentration der die bei diesem Druck die beiden Azeotrope TBA/Wasser und SBA/Wasser verbindenden Destillationsgrenzlinie, und bei diesem Druck das Gemisch destillativ in einen SBA aufweisenden Strom und einen überwiegend TBA und Wasser aufweisenden Strom getrennt wird.

2. Verfahren nach Anspruch 1,
**dadurch gekennzeichnet,**
**dass** die Abtrennung des Wassers im ersten Schritt mittels einer Destillationskolonne erfolgt.

3. Verfahren nach Anspruch 1 oder 2,
**dadurch gekennzeichnet,**
**dass** das Destillationsverfahren im ersten Schritt bei einem Druck von 70000 bis 500000 Pa (0,7 bis 5 bara) durchgeführt wird.

4. Verfahren nach Anspruch 3,
**dadurch gekennzeichnet,**
**dass** der Druck im zweiten Schritt auf 5000 bis kleiner 70000 Pa (0,05 bis kleiner 0,7 bara) eingestellt wird und die destillative Abtrennung des SBA bei diesem Druck erfolgt.

5. Verfahren nach Anspruch 3,
**dadurch gekennzeichnet,**
**dass** der Druck im zweiten Schritt auf größer 500000 bis 1500000 Pa (5 bis 15 bara) eingestellt wird und die destillative Abtrennung des SBA bei diesem Druck erfolgt.

6. Verfahren nach einem der Ansprüche 1 bis 5,
**dadurch gekennzeichnet,**
**dass** das im ersten Schritt erhaltene Destillat im zweiten Schritt destillativ in eine 2-Butanol aufweisende Fraktion getrennt wird, die weniger als 2 Massen-% tert.-Butanol aufweist.

7. Verfahren nach einem der Ansprüche 1 bis 6,
**dadurch gekennzeichnet,**
**dass** ein Teil des im zweiten Schritt erhaltenen Destillats mit dem im ersten Schritt erhaltenen Destillat als Zulauf in die destillative Auftrennung des zweiten Schritts eingesetzt wird.

8. Verfahren nach einem der Ansprüche 1 bis 7,
**dadurch gekennzeichnet,**
**dass** das 2-Butanol aus der Dampfphase des Verdampfers einer im zweiten Schritt eingesetzten Kolonne dampfförmig oder flüssig als Seitenstrom im Abtriebsteil dieser Kolonne abgenommen wird.

## Claims

1. Process for separating 2-butanol (SBA) from an industrial mixture which comprises 2-butanol, tert-butanol (TBA) and water and in which the proportion by mass of water is greater than the limit concentrations of the distillation boundary line connecting the two azeotropes TBA/water and SBA/water, **characterized in that**, in a first step, water is separated off from the mixture by means of a distillation process in such an amount that the distillate obtained has, in terms of its SBA/TBA/water composition, a proportion by mass of water which at a predetermined pressure is just greater than the limit concentration of the distillation boundary line connecting the two azeotropes TBA/water and SBA/water and, in a second step, the pressure is subsequently changed so that the mixture has, in terms of its SBA/TBA/water composition, a proportion by mass of water which at a predetermined pressure is less than the limit concentration of the distillation boundary line connecting the two azeotropes TBA/water and SBA/water at this pressure and the mixture is separated by distillation at this pressure into a stream comprising SBA and a stream comprising predominantly TBA and water.

2. Process according to Claim 1, **characterized in that** the removal of water in the first step is carried out by means of a distillation column.

3. Process according to Claim 1 or 2, **characterized in that** the distillation process in the first step is carried out at a pressure of from 70,000 to 500,000 Pa (0.7 to 5 bara).

4. Process according to Claim 3, **characterized in that** the pressure in the second step is set to from 5000 to 70,000 Pa (0.05 to < 0.7 bara) and the removal of the SBA by distillation is carried out at this pressure.

5. Process according to Claim 3, **characterized in that** the pressure in the second step is set to from > 500,000 to 1,500,000 Pa (5 to 15 bara) and the removal of the SBA by distillation is carried out at this pressure.

6. Process according to any of Claims 1 to 5, **characterized in that** the distillate obtained in the first step is separated by distillation in the second step to give a 2-butanol-containing fraction containing less than 2% by mass of tert-butanol.

7. Process according to any of Claims 1 to 6, **characterized in that** part of the distillate obtained in the second step together with the distillate obtained in the first step is used as feed to the fractional distillation of the second step.

8. Process according to any of Claims 1 to 7, **characterized in that** the 2-butanol is taken off from the vapor phase of the vaporizer of a column used in the second step in gaseous or liquid form as side stream in the stripping section of this column.

## Revendications

1. Procédé pour la séparation de 2-butanol (SBA) d'un mélange technique, qui présente du 2-butanol, du tert-butanol (TBA) et de l'eau, la proportion en masse d'eau dans le mélange étant supérieure aux concentrations limites de la ligne limite de distillation reliant les deux azéotropes TBA/eau et SBA/eau, **caractérisé en ce que** dans une première étape, on sépare du mélange, à l'aide d'un procédé de distillation, une quantité d'eau telle que le distillat obtenu présente, en ce qui concerne sa composition SBA/TBA/eau, à une pression prédéfinie, une proportion massique d'eau qui est justement encore supérieure à la concentration limite de la ligne limite de distillation reliant les deux azéotropes TBA/eau et SBA/eau et, dans une deuxième étape, on modifie ensuite la pression de manière telle que le mélange présente, en ce qui concerne sa composition SBA/TBA/eau à une pression prédéfinie, une proportion massique d'eau qui est inférieure à la concentration limite de la ligne limite de distillation reliant à cette pression les deux azéotropes TBA/eau et SBA/eau et le mélange est séparé à cette pression, par distillation, en un flux présentant du SBA et un flux présentant principalement du TBA et de l'eau.

2. Procédé selon la revendication 1, **caractérisé en ce que** la séparation de l'eau dans la première étape a lieu au moyen d'une colonne de distillation.

3. Procédé selon la revendication 1 ou 2, **caractérisé en ce que** le procédé de distillation dans la première étape est effectué à une pression de 70 000 à 500 000 Pa (0,7 à 5 bara).

4. Procédé selon la revendication 3, **caractérisé en ce que** la pression dans la deuxième étape est réglée à 5000 jusqu'à moins de 70 000 Pa (0,05 à moins de 0,7 bara) et la séparation par distillation du SBA a lieu à cette pression.

5. Procédé selon la revendication 3, **caractérisé en ce que** la pression dans la deuxième étape est réglée à plus de 500 000 jusqu'à 1 500 000 Pa (5 à 15 bara) et la séparation par distillation du SBA a lieu à cette pression.

6. Procédé selon l'une quelconque des revendications 1 à 5, **caractérisé en ce que** le distillat obtenu dans la première étape est séparé par distillation dans la deuxième étape en une fraction présentant du 2-butanol qui présente moins de 2% en masse de tert-butanol.

7. Procédé selon l'une quelconque des revendications 1 à 6, **caractérisé en ce qu'**une partie du distillat obtenu dans la deuxième étape est utilisée avec le distillat obtenu dans la première étape comme alimentation dans la séparation par distillation de la deuxième étape.

8. Procédé selon l'une quelconque des revendications 1 à 7, **caractérisé en ce que** le 2-butanol est prélevé de la phase vapeur de l'évaporateur d'une colonne utilisée dans la deuxième étape, sous forme de vapeur ou liquide comme flux latéral dans la partie d'épuisement de cette colonne.
